# EUROPÄISCHE PATENTSCHRIFT

(11) **EP 2 392 274 B1**
(45) Veröffentlichungstag und Bekanntmachung des Hinweises auf die Patenterteilung: **31.07.2019**
(21) Anmeldenummer: 11004285.0
(22) Anmeldetag: 25.05.2011
(51) Int. Cl.: A61B 17/34, A61F 9/007

(54) **Vorrichtung zum Einführen eines Mediums oder eines Instruments in den menschlichen Körper**
Device for inserting a medium or an instrument into the human body
Dispositif d'introduction d'un milieu ou d'un instrument dans le corps humain

(30) Priorität: 01.06.2010 DE 102010022403
(43) Veröffentlichungstag der Anmeldung: 07.12.2011
(73) Patentinhaber: Geuder AG, 69126 Heidelberg (DE)
(72) Erfinder: Geuder, Volker, 69126 Heidelberg (DE); Schleimer, Bernd, 69136 Heidelberg (DE); Draheim, René, 69207 Sandhausen (DE); Hattenbach, Lars-Olof, 67061 Ludwigshafen (DE); Frauenfeld, Dieter, 69121 Heidelberg (DE)
(74) Vertreter: Patent- und Rechtsanwälte Ullrich & Naumann

(56) Entgegenhaltungen:
- EP-A1- 1 886 653
- EP-A1- 2 138 117
- WO-A2-2007/035473
- US-A1- 2007 276 191

## Beschreibung

Die Erfindung betrifft eine Vorrichtung zum Einführen eines Mediums oder eines Instruments in den menschlichen Körper, insbesondere in das menschliche Auge, mit einem Anschlusskörper, einem im Anschlusskörper ausgebildeten Durchgang und einer sich an den Durchgang anschließenden Sonde, wobei die Sonde zum Einführen in den Körper und der Anschlusskörper auf der der Sonde zugewandten Seite zur Anlage außerhalb des Körpers und auf der der Sonde abgewandten Seite zum Ankoppeln eines Schlauchs, Instruments oder dgl. dient.

Vorrichtungen der gattungsbildenden Art, insbesondere zur Anwendung in der Ophthalmologie, sind unter dem Begriff Inzisionsvorrichtung oder Trokar hinlänglich aus der Praxis bekannt. Lediglich beispielhaft sei dazu auf die EP 1 886 653 A1 verwiesen.

Grundsätzlich geht es um eine Vorrichtung zum Einführen eines Mediums oder eines Instruments in den menschlichen Körper. Trokare sind hier lediglich beispielhaft genannt. Die Erfindung ist jedoch keineswegs auf Trokare eingeschränkt. Diese werden nachfolgend lediglich beispielhaft erörtert.

Wie bereits zuvor ausgeführt, handelt es sich bei der hier in Rede stehenden Vorrichtung um ein chirurgisches Instrument, welches beispielsweise für chirurgische Eingriffe im Auge eingesetzt wird. Im Rahmen entsprechender Eingriffe in das menschliche Auge werden regelmäßig mehrere Trokare durch die Sklera hindurch in das Auge eingeführt. Üblicherweise wird ein als Führungsrohr dienender erster Trokar mittels besonderer Lanze derart in das Auge eingesetzt, dass die Spitze der Sonde des Trokars bis in den Glaskörperraum des Auges reicht. Das von der Sonde abgewandte Ende des Trokars, das heißt der Anschlusskörper, dient auf der der Sonde abgewandten Seite zum Anschließen eines Schlauchs, über den das Innere des Auges mit einer Infusionsflüssigkeit versorgbar ist. Dieser Trokar wird üblicherweise auch als Infusionstrokar bezeichnet.

Des Weiteren ist es üblich, ein oder zwei weitere Trokare in das Auge einzusetzen, über die die benötigten Instrumente, beispielsweise Ultraschallschneidinstrumente oder Beleuchtungseinrichtungen, in das Auge einführbar sind. Diese Trokare werden üblicherweise als Instrumententrokare oder - entsprechend der Anwendung - als Beleuchtungstrokare bezeichnet.

Die hier in Rede stehenden Trokare haben den Vorteil, dass diese in eine kleine Öffnung der Sklera eingesetzt werden, wobei die Instrumente durch den jeweiligen Trokar hindurch austauschbar sind. Eine weiterreichende Reizung oder Beschädigung der Sklera wird durch Verwendung des Trokars vermieden.

Die gattungsbildende Vorrichtung ist jedoch insoweit problematisch, als der Anschlusskörper einen recht voluminösen Kopfbereich umfasst, der sich außerhalb des menschlichen Körpers bzw. des Auges befindet. Aufgrund seines Volumens bzw. aufgrund der Baugröße des Anschlusskörpers behindert dieser den operierenden Arzt und besteht die Gefahr, dass der Trokar bei der Handhabung anderer Trokare oder Instrumente aus dem Körper bzw. Auge - ungewollt - herausgezogen wird. Außerdem deckt der Anschlusskörper einen nicht unerheblichen Bereich um die eigentliche Inzision herum ab, so dass auch insoweit eine Behinderung stattfindet. Schließlich umfasst der recht groß bzw. voluminös bauende Anschlusskörper eine erhebliche Masse, die ein ungewolltes Herausrutschen der Sonde aus dem menschlichen Körper bzw. aus dem Auge begünstigt.

Der vorliegenden Erfindung liegt daher die Aufgabe zugrunde, eine gattungsbildende Vorrichtung, insbesondere einen Trokar, derart auszugestalten und weiterzubilden, dass sie/er sich sicher in einen menschlichen Körper bzw. in das Auge einsetzten lässt, wobei der Trokar die sonstige Handhabung des Operateurs nicht behindern soll.

Die voranstehende Aufgabe wird erfindungsgemäß durch eine Vorrichtung mit den Merkmalen des Patentanspruchs 1 gelöst. Danach ist die gattungsbildende Vorrichtung dadurch gekennzeichnet, dass die der Sonde abgewandte Seite des Anschlusskörpers konvex ausgebildet ist. Erfindungsgemäß ist erkannt worden, dass es - entgegen der gefestigten Meinung der Fachwelt - möglich ist, den Anschlusskörper der gattungsbildenden Vorrichtung bzw. des bekannten Trokars so flach wie möglich auszugestalten, jedenfalls so flach, dass er den Operateur bei der Handhabung der sonstigen Instrumente, beispielsweise anderer Trokare oder durch den Trokar in das Auge zu führender Instrumente, nicht stört. Durch die flache Ausgestaltung des Anschlusskörpers bietet dieser so gut wie keine Angriffsfläche gegenüber anderen Instrumenten oder gegenüber dem Operateur, so dass ein ungewolltes Herausstreifen des Trokars nahezu ausgeschlossen ist. Ganz im Gegenteil legt sich der Trokar mit seinem Anschlusskörper möglichst eng bzw. dicht an die Oberfläche des Auges, behindert jedenfalls in keiner Weise aufgrund eines herausragenden, eine Angriffsfläche bietenden Kopfsbereichs.

Im Konkreten ist es denkbar, dass der Anschlusskörper scheibenartig, tellerartig oder knopfartig ausgebildet ist. Anschlussseitig kann der Anschlusskörper abgerundet und anlageseitig flach oder leicht gewölbt bzw. konkav ausgebildet sein, insbesondere dann, wenn es sich bei der Vorrichtung um einen Trokar zum Einsatz am/im menschlichen Auge handelt. So kann sich der Anschlusskörper eng an die Oberfläche des Auges anschmiegen, und ragt dabei kaum vom Auge ab.

Insbesondere zur Begünstigung des Einführens eines Instruments durch die Vorrichtung bzw. durch den Trokar, beispielsweise eines Instruments mit langer Sonde, ist im Kopfbereich des flachen Anschlusskörpers eine trichterartige Öffnung vorgesehen, die in den Durchgang mündet. Das Einschieben eines lanzenartigen Instruments ist dadurch ganz erheblich begünstigt. Außerdem bietet der Trichter die Möglichkeit, unmittelbar an die Vorrichtung einen Schlauch anzukoppeln, nämlich mit einem entsprechend gestalteten Gegenstück.

Zur Ankopplung eines Instruments, insbesondere eines Schlauchs unmittelbar an den Anschlusskörper, ist im Durchgang, vorzugsweise unmittelbar hinter der trichterartigen Öffnung, eine Nut oder eine Wulst vorgesehen, die zur Bildung eines Snap-in-Mechanismus mit dem anzuschließenden Schlauch, Instrument oder dgl. dient. Mit anderen Worten ist am Schlauch oder am sonstigen Instrument eine komplementäre Ausgestaltung vorgesehen, die gemeinsam mit der Nut oder der Wulst im Anschlusskörper eine Snap-in-Verbindung bildet. Der unmittelbare Anschluss eines Schlauchs an den Anschlusskörper ist damit möglich.

In weiter vorteilhafter Weise ist im Durchgang, vorzugsweise im Übergangsbereich zur Sonde, eine zur Abdichtung dienende Dichtung, beispielsweise in Form einer Membrane, ausgebildet, die einfach oder kreuzförmig geschlitzt sein kann. Eine zum Durchstechen dienende Perforation kann dort alternativ oder ergänzend vorgesehen sein. Jedenfalls dient die Membrane zur Abdichtung, insbesondere dann, wenn ein einmal eingestecktes Instrument wieder aus der Sonde des Trokars herausgezogen wird, um nämlich dem Augeninnendruck entgegen zu wirken. Eine randseitige Abdichtung gegenüber einem eingeschobenen Instrument ist dadurch ebenfalls realisiert.

In Bezug auf eine sichere Fixierung der Vorrichtung bzw. des Trokars im Körper bzw. im Auge ist es von weiterem Vorteil, wenn die sich an den Anschlusskörper anschließende Sonde zumindest in einem an den Anschlusskörper angrenzenden Fixierungsbereich eine raue, genoppte oder im wesentlichen quer oder schräg zur Längsachse der Sonde gewellte bzw. geriefte Oberfläche aufweist. Alternativ ist es denkbar, dass im Falle einer Ausführung der Sonde als Drehteil diese im Fixierungsbereich Drehriefen umfasst. Auch ist es denkbar, dass der Fixierungsbereich durch ein Außengewinde der Sonde gebildet ist.

Bei dem Fixierungsbereich handelt es sich um einen funktionalen Bereich der Sonde nahe am Anschlusskörper. Grundsätzlich würde es ausreichen, beispielsweise bei der Anwendung der Vorrichtung als Trokar im menschlichen Auge, den Fixierungsbereich der Dicke der Sklera anzupassen oder diesen geringfügig länger auszugestalten. Jedenfalls ist es von Vorteil, wenn der Fixierungsbereich im oberen, dem Anschlusskörper nahen Drittel der Sonde oder aber auch über die erste Hälfte der Sonde hinweg ausgebildet ist.

Grundsätzlich ist es denkbar, dass es sich bei der Sonde um ein separates Bauteil handelt, welches mit dem Anschlusskörper mechanisch oder adhäsiv verbunden ist. In ganz besonders vorteilhafter Weise handelt es sich bei der Sonde um einen integralen Bestandteil des Anschlusskörpers, so dass sich eine spitzgusstechnische Herstellung der gesamten Vorrichtung anbietet.

In weiter vorteilhafter Weise ist der Anschlusskörper auf der der Sonde zugewandten Seite mit einer besonderen Anlagefläche ausgestattet, die zur Anlage außerhalb des Körpers, beispielsweise an die Außenfläche der Sklera, dient. Diese Außenfläche könnte mit einer rauen, genoppten, gerieften oder sonst wie strukturierten Oberfläche ausgestattet sein, so dass bei einer einmal erreichten Anlage am Auge bzw. an der Sklera eine Art Drehsicherung realisiert ist.

Die hier in Rede stehende Vorrichtung wird beispielsweise als sogenannter Beleuchtungstrokar verwendet. In besonders vorteilhafter Weise ist es möglich, die Vorrichtung bzw. den Trokar selbst als Lichtleiter auszugestalten, wonach zumindest ein Teil des Anschlusskörpers und die Sonde - oder ein Teil der Sonde oder ein Bereich entlang der Mantelfläche der Sonde, als Lichtleiter ausgebildet ist oder einen Lichtleiter mit entsprechender Durchführung umfasst. Im Rahmen einer solchen Ausgestaltung könnte unmittelbar an den Trokar, irgendwo im Bereich des Anschlusskörpers, Licht an- bzw. eingekoppelt werden und könnte der Durchgang für sonstige Instrumente oder zum Einleiten einer Flüssigkeit genutzt werden. Dabei ist es denkbar, dass der Anschlusskörper zumindest bereichsweise oder insgesamt durchsichtig ausgeführt ist, wodurch die Handhabung abermals begünstigt ist.

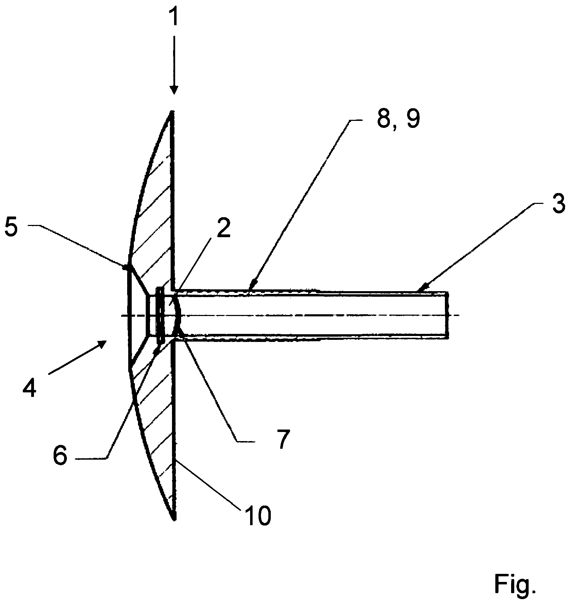

Es gibt nun verschiedene Möglichkeiten, die Lehre der vorliegenden Erfindung in vorteilhafter Weise auszugestalten und weiterzubilden. Dazu ist einerseits auf die dem Patentanspruch 1 nachgeordneten Patentansprüche und andererseits auf die nachfolgende Erläuterung eines Ausführungsbeispiels der Erfindung anhand der Zeichnung zu verweisen. In Verbindung mit der Erläuterung des bevorzugten Ausführungsbeispiels der Erfindung anhand der Zeichnung werden auch im Allgemeinen bevorzugte Ausgestaltungen und Weiterbildungen der Lehre erläutert. In der Zeichnung zeigt die
einzige Fig. in einer schematischen Ansicht, geschnitten, ein Ausführungsbeispiel eines in das menschliche Auge einzusetzenden Trokars.

Bei der gezeigten erfindungsgemäßen Vorrichtung handelt es sich um einen Trokar, der mittels Lanze durch die Sklera hindurch in das menschliche Auge eingesetzt wird.

Der in der einzigen Fig. - schematisch - gezeigte Trokar, ein Ausführungsbeispiel einer erfindungsgemäßen Vorrichtung, umfasst einen Anschlusskörper 1, einen im Anschlusskörper 1 ausgebildeten Durchgang 2 und eine sich an den Durchgang 2 anschließende Sonde 3. Die Sonde 3 dient zum Einstecken in den menschlichen Körper bzw. in das Auge. Der Anschlusskörper 1 dient auf der der Sonde 3 zugewandten Seite zur Anlage außerhalb des Auges, nämlich zur Anlage an der Sklera. Auf der der Sonde 3 abgewandten Seite, d. h. anschlussseitig, dient der Anschlusskörper 1 zum Ankoppeln eines in der Figur nicht gezeigten Schlauchs, zum Anstecken oder Einstecken eines Instruments, etc., wodurch dort eine Art Port gebildet ist.

Die einzige Figur zeigt deutlich, dass der Anschlusskörper 1 äußerst flach ausgebildet ist, nämlich kaum eine Bauhöhe aufweist. Im Konkreten ist der Anschlusskörper 1 ähnlich einem runden Schraubenkopf gebildet, nämlich scheibenartig, tellerartig oder knopfartig ausgebildet. Anschlussseitig ist der Anschlusskörper 1 abgerundet und anlageseitig flach ausgebildet, wobei der Anschlusskörper 1 anlageseitig auch gewölbt oder konkav ausgebildet sein kann, nämlich zur Begünstigung der Anlage an der Oberfläche des Auges.

Der bereits zuvor erwähnte Port 4 ist durch eine trichterartige Öffnung 5 gebildet, die in den Durchgang 2 mündet. Die trichterartige Öffnung 5 begünstigt das Einführen einer Sonde, Lanze oder dgl.. Ebenso begünstigt die trichterartige Öffnung 5 die unmittelbare Ankopplung des Anschlussstückes eine Schlauchs.

Das Ankoppeln eines Schlauchs oder eines sonstigen Instruments ist des Weiteren durch eine hinter der trichterartigen Öffnung 5 ausgebildete Nut 6 begünstigt, wodurch sich ein Snap-in-Mechanismus mit dem Anschlussstück eines anzuschließenden Schlauchs bilden lässt. Jedenfalls wirken die trichterartige Öffnung 5 und die Nut 6 zum Anschluss eines geeigneten Anschlussstücks zusammen. Eine einfache Ankopplung im Sinne eines Snap-in-Mechanismus ist dadurch realisiert.

Die einzige Figur lässt des Weiteren erkennen, dass im Durchgang 2, vorzugsweise im Übergangsbereich zur Sonde 3, eine zur Abdichtung dienende Membrane 7 ausgebildet ist, die beim Durchschieben eines lanzenartigen Instruments geöffnet wird. Beim Herausziehen des Instruments ist ein hinreichend guter Verschluss geschaffen, um bei einer Druckdifferenz zwischen dem Augeninneren und der Umgebung ein Heraustreten von Flüssigkeit aus dem Auge wirksam zu vermeiden.

In ganz besonders vorteilhafter Weise weist die Sonde 3 in einem an den Anschlusskörper 1 angrenzenden Fixierungsbereich 8 eine geriefte Oberfläche 9 auf, wobei es sich hier im Konkreten um Drehrillen handelt. Die Drehrillen bzw. Riefen bilden eine hinreichende Rauhigkeit, um ein ungewolltes Herausgleiten der Sonde 3 aus dem Randbereich der Sklera vermeiden zu können.

In der einzigen Fig. ist des Weiteren angedeutet, dass sich der Fixierungsbereich 8 unmittelbar an den Anschlusskörper 1 anschließt und sich um etwas mehr als die Hälfte der Länge der Sonde 3 erstreckt. Eine kürzere Ausführung des Fixierungsbereichs 8 ist denkbar, je nach konkreter Anwendung.

Des Weiteren ist in der einzigen Fig. angedeutet, dass auf der der Sonde 3 zugewandten Seite des Anschlusskörpers 1 eine Anlagefläche 10 vorgesehen ist, die ebenfalls mit einer rauen, genoppten, gerieften oder sonst wie strukturierten Oberfläche ausgestattet ist. Die Anlagefläche 10 dient zur weiterreichenden Fixierung, nämlich in Bezug auf eine ungewollte Drehung des Trokars 1.

In Bezug auf Merkmale, die sich der Figur nicht entnehmen lassen, sei zur Vermeindung von Widerholungen auf den allgemeinen Teil der Beschreibung verwiesen.

Schließlich sei angemerkt, dass das voranstehend erörterte Ausführungsbeispiel lediglich der beispielhaften Erörterung der erfindungsgemäßen Vorrichtung dient, diese jedoch nicht auf das Ausführungsbeispiel einschränkt.

### Bezugszeichenliste

- 1: Anschlusskörper
- 2: Durchgang
- 3: Sonde
- 4: Port
- 5: trichterartige Öffnung
- 6: Nut
- 7: Membrane
- 8: Fixierungsbereich
- 9: geriefte Oberfläche
- 10: Anlagefläche

## Patentansprüche

1. Vorrichtung zum der Einführen eines Mediums oder eines Instruments in das menschliche Auge (3), mit einem Anschlusskörper (1), einem im Anschlusskörper (1) ausgebildeten Durchgang (2) und einer sich an den Durchgang (2) anschließenden Sonde (3), wobei die Sonde (3) zum Einführen in das Auge und der Anschlusskörper (1) auf der der Sonde (3) zugewandten Seite zur Anlage außerhalb des Körpers und auf der der Sonde (3) abgewandten Seite zum Ankoppeln eines Schlauchs oder eines Instruments dient, und wobei der Anschlusskörper (1) flach, d.h. scheibenartig, tellerartig oder knopfartig, ausgebildet ist,
**dadurch gekenzeichnet**, dass die der Sonde (3) abgewandte Seite des Anschlusskörpers (1) konvex ausgebildet ist.

2. Vorrichtung nach Anspruch, **dadurch gekennzeichnet, dass** der Anschlusskörper (1) anschlussseitig abgerundet und anlageseitig eben/flach oder leicht gewölbt/konkav ausgebildet ist.

3. Vorrichtung nach Anspruch 1 oder 2, **dadurch gekennzeichnet, dass** im Anschlusskörper (1) eine trichterartige Öffnung (5) in den Durchgang (2) mündet.

4. Vorrichtung nach einem der Ansprüche 1 bis 3, **dadurch gekennzeichnet, dass** im Durchgang (2) eine Nut (6) oder eine Wulst zur Bildung eines Snap-in-Mechanismus mit dem anzuschließenden Schlauch, Instrument oder dgl. ausgebildet ist.

5. Vorrichtung nach einem der Ansprüche 1 bis 4, **dadurch gekennzeichnet, dass** im Durchgang (2), vorzugsweise im Übergangsbereich zur Sonde (3), eine zur Abdichtung dienende Membrane (7) ausgebildet ist.

6. Vorrichtung nach einem der Ansprüche 1 bis 5, **dadurch gekennzeichnet, dass** die Sonde (3) zumindest in einem an den Anschlusskörper (1) angrenzenden Fixierungsbereich (8) eine raue, genoppte oder im wesentlichen quer oder schräg zur Längsachse gewellte bzw. geriefte Oberfläche (9) aufweist,
oder dass zumindest die Sonde (3) als Drehteil ausgeführt ist und der Fixierungsbereich (8) durch Drehriefen gebildet ist,
oder dass der Fixierungsbereich (8) durch ein Außengewinde der Sonde (3) gebildet ist.

7. Vorrichtung nach einem der Ansprüche 1 bis 6, **dadurch gekennzeichnet, dass** die Sonde (3) integraler Bestandteil des Anschlusskörpers (1) ist.

8. Vorrichtung nach einem der Ansprüche 1 bis 7, **dadurch gekennzeichnet, dass** der Anschlusskörper (1) auf der der Sonde (3) zugewandten Seite eine zur Anlage außerhalb des Körpers dienende Anlagefläche (10) aufweist, die mit einer rauen, genoppten, gerieften oder sonst wie strukturierten Oberfläche (9) ausgestattet ist.

9. Vorrichtung nach einem der Ansprüche 1 bis 8, **dadurch gekennzeichnet, dass** der Anschlusskörper (1) zumindest bereichsweise oder insgesamt durchsichtig ausgeführt ist.

10. Vorrichtung nach einem der Ansprüche 1 bis 9, **dadurch gekennzeichnet, dass** zumindest ein Teil des Anschlusskörpers (1) und die Sonde (3) als Lichtleiter ausgebildet sind oder einen Lichtleiter umfassen.

## Claims

1. Device for introducing a medium or an instrument into the human eye (3), having a connection member (1), a passage (2) which is formed in the connection member (1) and a probe (3) which is connected to the passage (2), wherein the probe (3) is used for introduction into the eye and the connection member (1) is used at the side facing the probe (3) for abutment outside the body and at the side facing away from the probe (3) for coupling a hose or an instrument, and wherein the connection member (1) is constructed to be flat, that is to say, disc-like, plate-like or button-like,
**characterised in that** the side of the connection member (1) facing away from the probe (3) is constructed in a convex manner.

2. Device according to claim 1, **characterised in that** the connection member (1) is constructed at the connection side in a rounded manner and is constructed at the abutment side in a planar/flat or slightly curved/concave manner.

3. Device according to claim 1 or 2, **characterised in that** in the connection member (1) a funnel-shaped opening (5) opens in the passage (2).

4. Device according to any one of claims 1 to 3,
**characterised in that** in the passage (2) a groove (6) or a bead for forming a snap-in mechanism with the hose, instrument or the like which is intended to be connected is formed.

5. Device according to any one of claims 1 to 4,
**characterised in that** in the passage (2), preferably in the transition region to the probe (3), a membrane (7) which is used for sealing is formed.

6. Device according to any one of claims 1 to 5,
**characterised in that** the probe (3) at least in a fixing region (8) adjacent to the connection member (1) has a surface (9) which is rough, burled or which is undulated or grooved substantially transversely or obliquely relative to the longitudinal axis,
or **in that** at least the probe (3) is constructed as a rotary component and the fixing region (8) is formed by machining grooves,
or **in that** the fixing region (8) is formed by means of an outer thread of the probe (3).

7. Device according to any one of claims 1 to 6,
**characterised in that** the probe (3) is an integral component of the connection member (1).

8. Device according to any one of claims 1 to 7,
**characterised in that** the connection member (1) has at the side facing the probe (3) an abutment face (10) which is used for abutment outside the body, and which is provided with a rough, burled, grooved or otherwise structured surface (9).

9. Device according to any one of claims 1 to 8,
**characterised in that** the connection member (1) is constructed in an at least partially or completely transparent manner.

10. Device according to any one of claims 1 to 9,
**characterised in that** at least a portion of the connection member (1) and the probe (3) are constructed as a light guide or comprise a light guide.

## Revendications

1. Dispositif d'introduction d'un fluide ou d'un instrument dans l'oeil humain (3), avec un corps de raccordement (1), un passage (2) réalisé dans le corps de raccordement (1) et une sonde (3) se raccordant au passage (2), la sonde (3) permettant l'introduction dans l'oeil humain et le corps de raccordement (1), sur le côté orienté vers la sonde (3), permettant l'appui à l'extérieur du corps et sur le côté opposé à la sonde (3), permettant le couplage d'un tuyau ou d'un instrument, et le corps de raccordement (1) présentant une forme plate, c'est-à-dire, la forme d'un disque, d'un plateau ou d'un bouton,
**caractérisé en ce que** le côté, opposé à la sonde (3), du corps de raccordement (1) présente une forme convexe.

2. Dispositif selon la revendication, **caractérisé en ce que** le corps de raccordement (1) présente, du côté du raccordement, une forme arrondie et, du côté de l'appui, une forme plane/plate ou légèrement incurvée/concave.

3. Dispositif selon la revendication 1 ou 2, **caractérisé en ce que**, dans le corps de raccordement (1), une ouverture en forme d'entonnoir (5) débouche dans le passage (2).

4. Dispositif selon l'une des revendications 1 à 3, **caractérisé en ce que**, dans le passage (2), une rainure (6) ou un bourrelet est réalisé pour la formation d'un mécanisme d'encliquetage avec le tuyau, instrument ou autre qui doit y être raccordé.

5. Dispositif selon l'une des revendications 1 à 4, **caractérisé en ce que**, dans le passage (2), de préférence dans la zone de transition avec la sonde (3), est disposée une membrane (7) servant de joint d'étanchéité.

6. Dispositif selon l'une des revendications 1 à 5, **caractérisé en ce que** la sonde (3) présente, au moins dans une zone de fixation (8) adjacente au corps de raccordement (1), une surface (9) rugueuse, cannelée ou bien ondulée ou rainurée transversalement ou en oblique par rapport à l'axe longitudinal,
ou **en ce qu'**au moins la sonde (3) est réalisée comme une partie rotative et la zone de fixation (8) est formée par un striage rotatif,
ou **en ce que** la zone de fixation (8) est constituée d'un filetage externe de la sonde (3).

7. Dispositif selon l'une des revendications 1 à 6, **caractérisé en ce que** la sonde (3) fait partie intégrante du corps de raccordement (1).

8. Dispositif selon l'une des revendications 1 à 7, **caractérisé en ce que** le corps de raccordement (1) comprend, sur le côté orienté vers la sonde (3), une surface d'appui (10) permettant un appui à l'extérieur du corps, qui est munie d'une surface (9) rugueuse, cannelée, rainurée ou texturée d'une autre manière.

9. Dispositif selon l'une des revendications 1 à 8, **caractérisé en ce que** le corps de raccordement (1) est réalisé de manière transparente, au moins à certains endroits ou sur son intégralité.

10. Dispositif selon l'une des revendications 1 à 9, **caractérisé en ce qu'**au moins une partie du corps de raccordement (1) et la sonde (3) sont conçus comme des fibres optiques ou comprennent une fibre optique.
